# EUROPEAN PATENT APPLICATION

(11) **EP 2 800 019 A1**
(43) Date of publication of application: **05.11.2014**
(21) Application number: 12862944.1
(22) Date of filing: 09.08.2012
(51) Int. Cl.: G06F 17/50, G06F 19/00

(54) **ANALYSIS DEVICE AND SIMULATION METHOD**

(30) Priority: 26.12.2011 JP 2011283801
(71) Applicant: Sumitomo Heavy Industries, Ltd., Tokyo 141-6025 (JP)
(72) Inventor: OHNISHI, Yoshitaka, Yokosuka-shi Kanagawa 237-8555 (JP)
(74) Representative: Louis Pöhlau Lohrentz
(86) International application number: PCT/JP2012/005070
(87) International publication number: WO 2013/099052

(57) **Abstract**

An analysis device 100 includes a defined system acquisition unit 110 configured to acquire a system including a plurality of particles defined in a virtual space as a defined system, a numerical computation unit 114 configured to numerically compute a governing equation, which governs the motion of each particle, and to update the position of each particle based on the computation result, a particle group specification unit 128 configured to specify a particle group, the relative position relationship of which is substantially maintained before and after the position is updated, from a plurality of particles in the defined system, a system position adjustment unit 134 configured to, when a system obtained as a result of updating the position is referred to as an updated system, adjust the relative positions of the defined system and the updated system such that the specified particle group and a particle group in the updated system corresponding to the specified particle group overlap each other, and a position relationship change unit 140 configured to change the relative position relationship between a particle in the defined system and a particle in the updated system corresponding to the particle in the defined system for the defined system and the updated system having the adjusted relative positions.

## Description

### Technical Field

The present invention relates to an analysis device which analyzes a particle system, and a simulation method.

### Background Art

In the related art, as a method of investigating a phenomenon of a general material science using a computer based on classical mechanics, quantum mechanics, or the like, a simulation based on a molecular dynamics method (hereinafter, referred to as an MD method) or a renormalized molecular dynamics method (hereinafter, referred to as an RMD method), which is developed from the MD method to handle a system on macro scale, is known (for example, see PTL 1). The MD method or the RMD method is a kind of particle method which describes a simulation object by a collection of particles.

The particle method handles a dynamic phenomenon, such as a flow, as well as a static phenomenon, and is thus attracting attention as a simulation method, which replaces a method of the related art, such as a finite element method, which primarily has a static phenomenon as an object to be analyzed.

### Citation List

### Patent Literature

[PTL 1] Japanese Unexamined Patent Application Publication No. 2009-37334

### Summary of Invention

### Technical Problem

One of the purposes of the simulation is to understand how a system changes due to the simulation. When the amount of change is smaller than the dimension of the system, or the like, it is difficult to understand a change form only by visually confirming the system before and after the simulation on a display.

Accordingly, for example, when a finite element method is used, it is thought that the amount of movement from an initial position of a node is deformed directly. In the finite element method, since parallel movement or rotation of a model before and after a simulation is not taken into consideration, it seems that, in many cases, a desired result can be obtained by the deformation.

However, in the particle method, computation is performed on an assumption that each particle changes in position while interacting with other particles. Accordingly, since it is often the case that the position or direction of the entire system before and after the simulation changes, in many cases, it is not visually correct if only the movement distance of the particle is deformed.

The invention has been accomplished in consideration of the above-described problem, and an object of the invention is to provide an analysis technique capable of readily capturing a phenomenon generated in a system in a simulation using a particle method.

### Solution to Problem

An aspect of the invention relates to an analysis device. The analysis device includes a defined system acquisition unit configured to acquire a system including a plurality of particles defined in a virtual space as a defined system, a numerical computation unit configured to numerically compute a governing equation, which governs the motion of each particle of the defined system, and to update the position of each particle of the defined system based on the computation result, a particle group specification unit configured to specify a particle group, the relative position relationship of which is substantially maintained before and after the update by the numerical computation unit, from a plurality of particles in the defined system, a system position adjustment unit configured to, when a system obtained as a result of the update by the numerical computation unit is referred to as an updated system, adjust the relative positions of the defined system and the updated system such that the particle group specified by the particle group specification unit and a particle group in the updated system corresponding to the specified particle group overlap each other, and a position relationship change unit configured to change the relative position relationship between a particle in the defined system and a particle in the updated system corresponding to the particle in the defined system for the defined system and the updated system having the relative positions adjusted by the system position adjustment unit.

According to this aspect, it is possible to adjust the relative positions of the defined system and the updated system based on the particle group specified by the particle group specification unit.

Another aspect of the invention is a simulation method. This method includes a step of acquiring a system including a plurality of particles defined in a virtual space as a defined system, a step of numerically computing a governing equation, which governs the motion of each particle of the defined system, a step of updating the position of each particle of the defined system based on the computation result, a step of, when a system obtained as a result of the update is referred to as an updated system, adjusting the relative positions of the defined system and the updated system, and a step of increasing the difference between the defined system and the updated system after the relative positions are adjusted.

It is to be noted that any arbitrary combination of the above-described structural components or rearrangement of the structural components and the expressions of the invention among a device, a method, a system, a computer program, a recording medium having a computer program recorded thereon, and the like are also effective as the aspects of the invention.

### Advantageous Effects of Invention

According to the invention, it is possible to readily capture a phenomenon generated in a system in a simulation using a particle method.

### Brief Description of Drawings

Fig. 1 is a schematic view showing an initial state before computation and a steady state after computation when a system having three particles defined in a virtual two-dimensional space is computed using a particle method.
Fig. 2 is a schematic view showing a state after parallel movement and rotation are carried out for a system in an initial state.
Fig. 3 is a schematic view showing a mode in which a particle moves to an end point of an enlarged difference vector obtained by multiplying a difference vector α times.
Fig. 4 is a block diagram showing the functions and configuration of an analysis device according to an embodiment.
Figs. 5(a) to 5(d) are representative screen diagrams showing an example of a simulation result by the analysis device.
Fig. 6 is a data structural diagram showing an example of a defined system retaining unit of Fig. 4.
Fig. 7 is a flowchart showing an example of a sequence of processing in the analysis device of Fig. 4.

### Description of Embodiments

Hereinafter, the same or similar structural components, members, and processing shown in the respective drawings are represented by the same reference numerals, and overlapping description will be appropriately omitted.

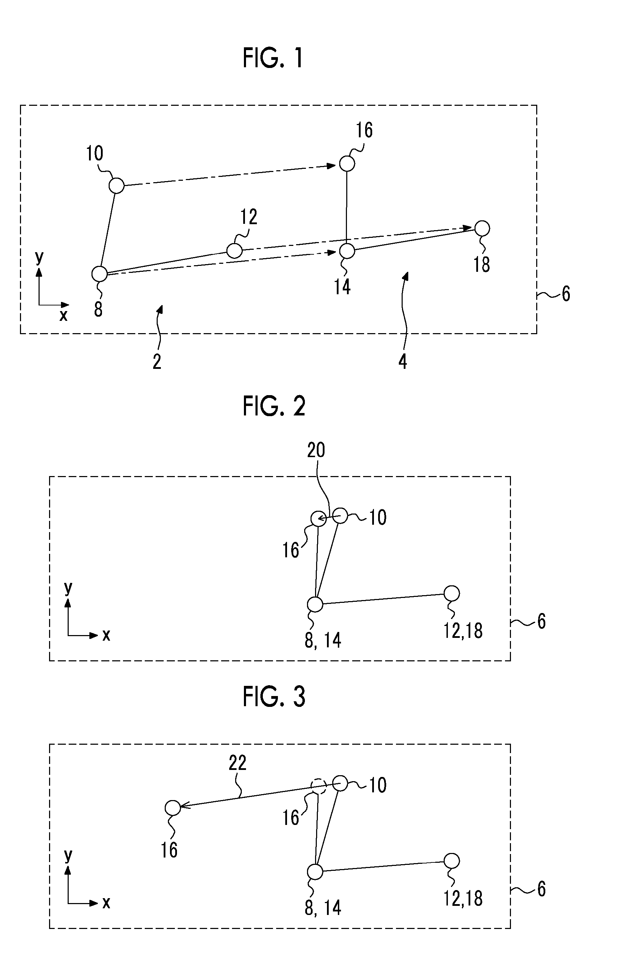

Fig. 1 is a schematic view showing an initial state 2 before computation and a steady state 4 after computation when a system having three particles defined in a virtual two-dimensional space 6 is computed using a particle method. With the computation using the particle method, particles 8, 10, and 12 in the initial state 2 respectively become particles 14, 16, and 18 in the steady state 4. The movement of each particle in this case is indicated by a one-dot-chain line in Fig. 1. The positions of the particles are all different between the initial state 2 and the steady state 4. In this way, in the computation by the particle method, unlike the finite element method or the like, the entire system often moves before and after computation.

The analysis device according to the embodiment compares the relative position relationship between the particles of the system in the initial state 2 with the relative position relationship between the particles of the system of in the steady state 4. In particular, the analysis device searches for a pair of particles in which the relative distance, that is, the inter-particle distance will not substantially change before and after computation. In the example shown in Fig. 1, it is assumed that the inter-particle distance between the particle 8 and the particle 12 will not substantially changed, that is, the inter-particle distance between the particle 8 and the particle 12 and the inter-particle distance between the particle 14 and the particle 18 are substantially the same.

The analysis device performs congruence transformation for the system in the initial state 2 such that the particle 8 and the particle 12 of the system in the initial state 2 respectively overlap the particle 14 and the particle 18 of the system in the steady state 4. For example, the analysis device moves the system in the initial state 2 in parallel such that the particle 8 overlaps the particle 14, and then rotates the system in the initial state 2 such that the particle 12 overlaps the particle 18.

Fig. 2 is a schematic view showing a state after parallel movement and rotation are carried out for the system in the initial state 2.

In the state where parallel movement and rotation are carried out for the system in the initial state 2, the analysis device obtains a difference vector 20 from the position of the particle 10 not used as a reference of congruence transformation to the position of the corresponding particle 16. The analysis device multiplies the difference vector 20 α (α > 1) times and moves the particle 16 to a position corresponding to the end point of the enlarged difference vector 20.

Fig. 3 is a schematic view showing a mode in which the particle 16 is moved to the end point of an enlarged difference vector 22 obtained by multiplying the difference vector 20 α times.

In this way, in the analysis device of this embodiment, a difference before and after computation by a particle method increases based on a predetermined reference portion in a system. In the particle method, while the entire system often moves by computation, when the method of this embodiment is used, it is possible to visualize deformation of the system with exaggeration after the movement of the entire system is absorbed by comparison of the reference portion.

In this embodiment, although a case where the particle system is analyzed using the MD method is described, it is obvious to those skilled in the art in contact with the specification that the technical idea of this embodiment can be applied to a case where the particle system is analyzed using other particle methods, such as renormalized molecular dynamics, DEM (Distinct Element Method), SPH (Smoothed Particle Hydrodynamics), and MPS (Moving Particle Semi-implicit).

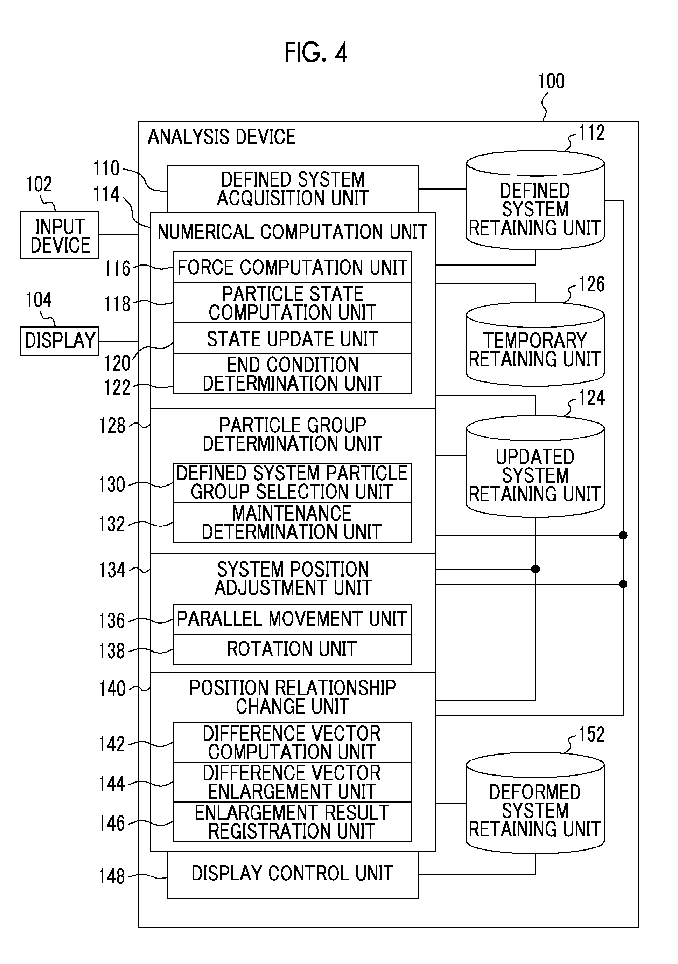

Fig. 4 is a block diagram showing the functions and configuration of the analysis device 100 according to this embodiment. Although respective blocks shown in the drawing can be realized by hardware, for example, an element, such as a CPU (central processing unit) of a computer, or a mechanical device, or may be realized by software, for example, a computer program or the like, functional blocks which are realized by collaboration of hardware and software are drawn. Accordingly, it is understood by those skilled in the art of contact with the specification that these functional blocks can be realized by combination of hardware and software in various ways.

The analysis device 100 is connected to an input device 102 and a display 104. The input device 102 may be a keyboard, a mouse, or the like which receives an input of a user related to processing executed on the analysis device 100. The input device 102 may be configured to receive an input from a network, such as Internet, or a recording medium, such as a CD or a DVD.

The analysis device 100 includes a defined system acquisition unit 110, a numerical computation unit 114, a particle group specification unit 128, a system position adjustment unit 134, a position relationship change unit 140, a display control unit 148, a defined system retaining unit 112, a temporary retaining unit 126, an updated system retaining unit 124, and a deformed system retaining unit 152.

The defined system acquisition unit 110 acquires a system including N (where N is a natural number) particles defined in a virtual space as a defined system P. The defined system P is defined as simulating a real object, such as a gear or a motor. In particular, the defined system acquisition unit 110 disposes N particles in the virtual space based on input information acquired from the user through the input device 102 and provides speed to each of the disposed particles. The defined system P is a system including the N particles defined in this way. The defined system acquisition unit 110 registers a particle ID for specifying each disposed particle, the position of the particle, and the speed of the particle in the defined system retaining unit 112 in association with one another.

A particle may correspond to a real-world atom or molecule. The defined system acquisition unit 110 may acquire data of the defined system P from the outside through a network or a recording medium and may register the acquired data in the defined system retaining unit 112.

Hereinafter, a case where all particles of the defined system P are set to have the same quality or equivalents, and a potential energy function is a pair potential and set to have the same shape regardless of particles will be described. However, it is obvious to those skilled in the art of contact with the specification that the technical idea of this embodiment can be applied to other cases.

The numerical computation unit 114 refers to the defined system retaining unit 112 and numerically computes a governing equation which governs the motion of each particle of the defined system P. In particular, the numerical computation unit 114 performs repetitive computation according to an equation of motion of discretized particles. The numerical computation unit 114 updates the position of each particle of the defined system P based on the computation result.

The numerical computation unit 114 includes a force computation unit 116, a particle state computation unit 118, a state update unit 120, and an end condition determination unit 122.

The numerical computation unit 114 duplicates data of the defined system P retained in the defined system retaining unit 112 to the temporary retaining unit 126 prior to starting computation. The numerical computation unit 114 updates the position and speed of each particle retained in the temporary retaining unit 126 with the position and speed of each particle duplicated from the defined system retaining unit 112 as initial values each time numerical computation is repeated. The update corresponds to the update of the position and speed of each particle of the defined system P. When ending numerical computation, the numerical computation unit 114 registers the position and speed of each particle retained in the temporary retaining unit 126 in the updated system retaining unit 124 as the position and speed of each particle of an updated system Q.

When numerical computation is continued until the system is placed in the steady state, the defined system P corresponds to the initial state of the system, and the updated system Q corresponds to the steady state of the system.

The force computation unit 116 refers to data retained in the temporary retaining unit 126 and computes a force applied to the particle based on the distance between the particles for each particle. For an i-th (1 ≤ i ≤ N) particle, the force computation unit 116 decides particles (hereinafter, referred to as near particles) which are at a distance from the i-th particle smaller than a predetermined cutoff distance. For each near particle, the force computation unit 116 computes the force applied to the i-th particle by the near particle based on the potential energy function between the near particle and the i-th particle and the distance between the near particle and the i-th particle. In particular, the force computation unit 116 calculates the force from the value of the gradient of the potential energy function in the value of the distance between the near particle and the i-th particle. The force computation unit 116 adds the force applied to the i-th particle by the near particle for all near particles to calculate the force applied to the i-th particle.

The particle state computation unit 118 refers to data retained in the temporary retaining unit 126 and applies the force computed by the force computation unit 116 to the equation of motion of discretized particles for each particle to compute at least one of the position and speed of the particle. In this embodiment, the particle state computation unit 118 computes both the position and speed of the particle.

The particle state computation unit 118 computes the speed of the particle from the equation of motion of discretized particles including the force computed by the force computation unit 116. The particle state computation unit 118 substitutes the force computed by the force computation unit 116 in the equation of motion of particles discretized using a predetermined minute time interval Δt based on a predetermined numerical analysis method, such as a leapfrog method or an Euler method, for the i-th particle, thereby computing the speed of the particle. In the computation, the speed of the particle computed in the previous repetitive computation cycle is used.

The particle state computation unit 118 calculates the position of the particle based on the computed speed of the particle. The particle state computation unit 118 applies the computed speed of the particle to a relationship expression of position and speed of discretized particles using the time interval Δt based on a predetermined numerical analysis method for the i-th particle, thereby computing the position of the particle. In the computation, the position of the particle computed in the previous repetitive computation cycle is used.

The state update unit 120 updates the position and speed of each particle retained in the temporary retaining unit 126 to the position and speed computed by the particle state computation unit 118.

The end condition determination unit 122 performs determination about whether or not to end repetitive computation in the numerical computation unit 114. An end condition for ending repetitive computation is, for example, that repetitive computation is performed a predetermined number of times, an end instruction is received from the outside, or the system reaches the steady state. When the end condition is satisfied, the end condition determination unit 122 ends repetitive computation in the numerical computation unit 114 and registers the position and speed of each particle retained in the temporary retaining unit 126 in the updated system retaining unit 124 as the position and speed of each particle of the updated system Q. Accordingly, the updated system Q is a system which is obtained as a result of updating the position and speed of each particle of the defined system P by the numerical computation unit 114. When the end condition is not satisfied, the end condition determination unit 122 returns the process to the force computation unit 116.

The particle group specification unit 128 specifies a particle group, the relative position relationship of which is substantially maintained before and after the update of the position by the numerical computation unit 114, from N particles in the defined system P. In particular, the particle group specification unit 128 specifies a particle group, the inter-particle distance of which is substantially maintained before and after the update of the position. When the virtual space in which the defined system P is defined is two-dimensional, the nearest particle pair (hereinafter, referred to as an inter-particle distance maintaining pair) is specified as a particle group, and when the virtual space is three-dimensional, three particles forming least triangular elements are specified as a particle group. Hereinafter, a case where the defined system P is defined in a two-dimensional virtual space will be described.

The particle group specification unit 128 includes a defined system particle group selection unit 130 and a maintenance determination unit 132.

The defined system particle group selection unit 130 refers to the defined system retaining unit 112 and selects a particle pair, in which one particle of the N particles in the defined system P is the nearest particle of another particle, that is, a particle nearest to another particle, as a candidate pair. The defined system particle group selection unit 130 computes the inter-particle distance of the selected candidate pair.

The maintenance determination unit 132 refers to the updated system retaining unit 124 and specifies a particle pair corresponding to the candidate pair selected by the defined system particle group selection unit 130 from the N particles in the updated system Q. In particular, the maintenance determination unit 132 specifies two particles of the updated system Q having the same particle IDs as the particle IDs of two particles in the candidate pair. The maintenance determination unit 132 computes the inter-particle distance of the specified particle pair. The maintenance determination unit 132 compares the computed inter-particle distance with the inter-particle distance of the candidate pair computed by the defined system particle group selection unit 130, when both coincide with each other within a predetermined error range, determines that the candidate pair is a pair in which the inter-particle distance is substantially maintained before and after the update of the position, and specifies the candidate pair as an inter-particle distance maintaining pair. The maintenance determination unit 132 specifies the particle pair of the updated system Q specified by the maintenance determination unit 132 as a corresponding pair.

When both do not coincide with each other, the maintenance determination unit 132 returns the process to the defined system particle group selection unit 130, and the defined system particle group selection unit 130 selects another candidate pair.

The system position adjustment unit 134 adjusts the relative positions of the defined system P and the updated system Q such that the inter-particle distance maintaining pair and the corresponding pair overlap each other. In particular, the system position adjustment unit 134 performs congruence transformation for at least one of the defined system P and the updated system Q to adjust the relative positions of the defined system P and the updated system Q. The congruence transformation includes transformation corresponding to parallel movement and transformation corresponding to rotation.

The system position adjustment unit 134 includes a parallel movement unit 136 and a rotation unit 138.

The parallel movement unit 136 moves the entire defined system P in parallel such that the position of one particle of the inter-particle distance maintaining pair is substantially the same as the position of one particle of the corresponding pair. The parallel movement may be performed by adding a predetermined parallel movement vector to the position vector of each particle of the defined system P. The parallel movement unit 136 updates the position of each particle retained in the defined system retaining unit 112 to the position of each particle obtained as a result of parallel movement.

The rotation unit 138 rotates the entire defined system P such that the position of the other particle of the inter-particle distance maintaining pair is substantially the same as the position of the other particle of the corresponding pair after the position of one particle of the inter-particle distance maintaining pair matches with the position of one particle of the corresponding pair by the parallel movement unit 136. The rotation may be performed by integrating a predetermined rotation matrix to the position vector of each particle of the defined system P after the update by the parallel movement unit 136. The rotation unit 138 updates the position of each particle retained in the defined system retaining unit 112 to the position of each particle obtained as a result of rotation.

The position relationship change unit 140 changes the relative position relationship between a particle in the defined system P and a particle in the updated system Q corresponding to the particle in the defined system P for the defined system P and the updated system Q having the relative positions adjusted by the system position adjustment unit 134. In particular, the position relationship change unit 140 changes the inter-particle distance between the particle in the defined system P and the particle in the updated system Q corresponding to the particle in the defined system P in a predetermined ratio.

The position relationship change unit 140 includes a difference vector computation unit 142, a difference vector enlargement unit 144, and an enlargement result registration unit 146.

The difference vector computation unit 142 refers to the defined system retaining unit 112 and the updated system retaining unit 124 after the update by the rotation unit 138 and computes a difference vector, which has the position retained in the defined system retaining unit 112 after the update by the rotation unit 138 as a start point and the position retained in the updated system retaining unit 124 as an end point, for each particle. Specifically, the difference vector computation unit 142 subtracts the position vector corresponding to the position retained in the defined system retaining unit 112 after the update by the rotation unit 138 from the position vector corresponding to the position retained in the updated system retaining unit 124 for each particle ID, thereby computing the difference vector.

The difference vector enlargement unit 144 multiplies the difference vector computed by the difference vector computation unit 142 by a ratio γ greater than 1, thereby computing an enlarged difference vector.

The enlargement result registration unit 146 adds the enlarged difference vector computed by the difference vector enlargement unit 144 to the position vector corresponding to the position retained in the defined system retaining unit 112 after the update by the rotation unit 138 for each particle ID, thereby obtaining a new position vector. The enlargement result registration unit 146 registers the particle ID and the position corresponding to the new position vector in the deformed system retaining unit 152 in association with each other.

The display control unit 148 displays a simulation result based on the position of each particle retained in the deformed system retaining unit 152 on the display 104.

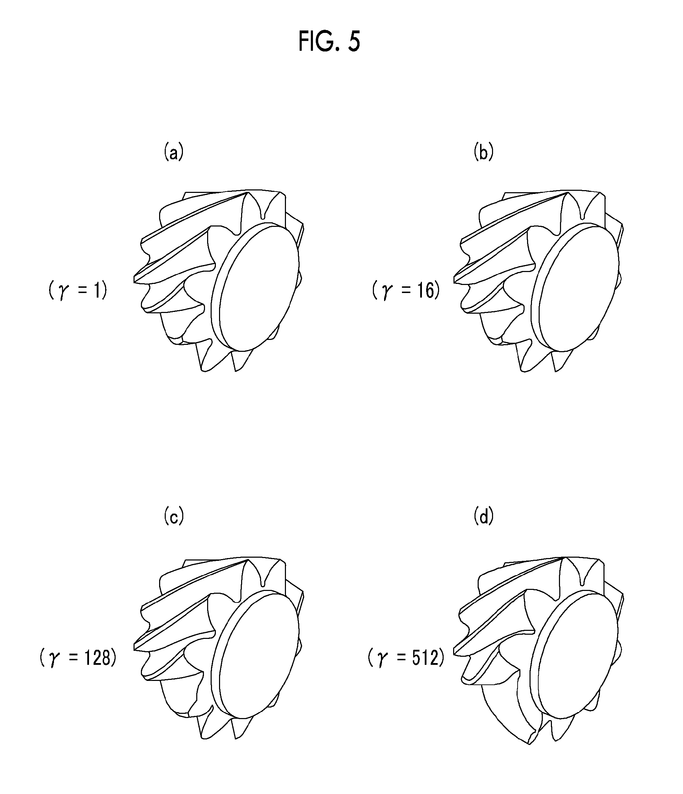

Figs. 5(a) to 5(d) are representative screen diagrams showing an example of a simulation result by the analysis device 100. In Figs. 5(a) to 5(d), one of meshed gears is simulated, and how the tooth surface of the gear is deformed by meshing is of interest.

Fig. 5(a) shows an updated system in which there is no change by the position relationship change unit 140, that is, an updated system corresponding to γ = 1. The difference between the defined system and the updated system is small with respect to the scale of Fig. 5(a), and the defined system is drawn in the same manner as Fig. 5(a) excluding the overall position of the system. Figs. 5(b), 5(c), and 5(d) respectively show updated systems corresponding to γ = 16, 128, and 512. It is understood that, as the ratio γ of deformation increases, it is possible to more easily understand visually how the tooth surface is deformed.

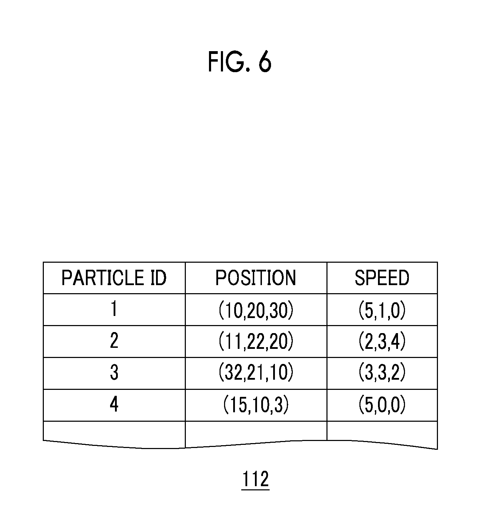

Fig. 6 is a data structural diagram showing an example of the defined system retaining unit 112. The defined system retaining unit 112 retains the particle ID, the position of the particle, and the speed of the particle in association with one another. The temporary retaining unit 126, the updated system retaining unit 124, and the deformed system retaining unit 152 have the same data structure as shown in Fig. 6.

In the above-described embodiment, an example of the retaining unit is a hard disk or a memory. It is understood by those skilled in the art of contact with the specification that the respective units can be realized by a CPU (not shown), a module of an installed application program, a module of a system program, a memory which temporarily stores the contents of data read from a hard disk, or the like based on the description of the specification.

The operation of the analysis device 100 having the above-described configuration will be described.

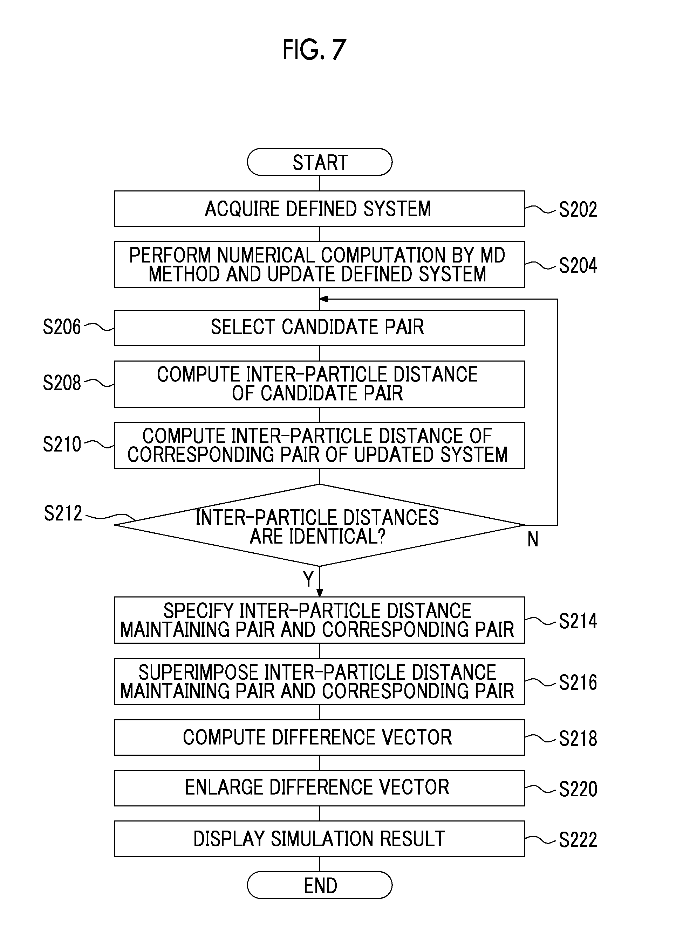

Fig. 7 is a flowchart showing an example of a sequence of processing in the analysis device 100. The defined system acquisition unit 110 acquires data representing the defined system P (S202). The numerical computation unit 114 performs numerical computation by the MD method for the defined system P and updates the defined system P based on the computation result (S204). The particle group specification unit 128 selects a candidate pair, which has not been selected before as a candidate pair from N particles in the defined system P before the numerical computation (S206). When there is no candidate pair to be selected, the particle group specification unit 128 may return an error. The particle group specification unit 128 computes the inter-particle distance of the candidate pair (S208). The particle group specification unit 128 computes the inter-particle distance of the particle pair of the updated system Q corresponding to the candidate pair (S210). The particle group specification unit 128 performs determination about whether or not the computed two inter-particle distances substantially coincide with each other (S212). When it is determined that two inter-particle distances do not coincide with each other (N in S212), the particle group specification unit 128 selects another candidate pair (S206). When it is determined that the two inter-particle distances coincide with each other (Y in S212), the particle group specification unit 128 specifies the candidate pair as an inter-particle distance maintaining pair and specifies the particle pair of the updated system Q corresponding to the candidate pair as a corresponding pair (S214). The system position adjustment unit 134 superimposes the inter-particle distance maintaining pair and the corresponding pair (S216). The position relationship change unit 140 computes the difference vector for each particle from the result of superimposition (S218). The position relationship change unit 140 enlarges the difference vector in a predetermined ratio (S220). The display control unit 148 displays a simulation result based on the enlarged difference vector on the display 104 (S222).

According to the analysis device 100 of this embodiment, it is possible to visualize change before and after numerical computation with exaggeration in a simulation using a particle method. The change is displayed with exaggeration (deformed), whereby, even when actual change is small, it is possible for the user to more accurately understand the change qualitatively.

For example, in general, it is difficult to understand how and where a result of a simulation is deformed even referring to Fig. 5 (a) or 5(b). However, referring to Fig. 5(c) or 5(d), it is possible to easily understand visually how and where the tooth surface is deformed. In this way, the method of this embodiment can be preferably used to visualize a structure which can be deformed when receiving a load.

In the analysis device 100 of this embodiment, the relative positions of the defined system and the updated system are adjusted before the difference between the defined system and the updated system is computed. With this, like a particle method, even when a simulation method is used, in which the overall position of the defined system and the overall position of the updated system are easily changed, it becomes possible to accurately compare the defined system and the updated system.

The inventors have recognized from the experience as those skilled in the art that, when a simulation is performed using a particle method, even if the overall position of the defined system and the overall position of the updated system are different, in most cases, there is a particle pair in which the inter-particle distance is not substantially changed before and after numerical computation. In this embodiment, based on the recognition of the inventors, an inter-particle distance maintaining pair is specified, and the defined system and the updated system are superimposed based on the inter-particle distance maintaining pair. Therefore, it is possible to increase applicability of the method of this embodiment.

The configuration and operation of the analysis device 100 according to the embodiment have been described. It is understood by those skilled in the art that the embodiment is just for illustration, and various modifications may be made to a combination of structural components or processing and still fall within the scope of the invention.

In the embodiment, although a case where both the position and speed of the particle are computed in the numerical computation unit 114 has been described, the invention is not limited thereto. For example, as a method for numerical analysis, like a Verlet method, a method in which, when computing the position of the particle, the position of the particle may be computed directly from the force applied to the particle, and the speed of the particle may not be computed explicitly is known, and the technical idea of this embodiment may be applied to this method.

In the embodiment, although a case where the particle group which is specified from the defined system P by the particle group specification unit 128 is the nearest particle pair has been described, the invention is not limited thereto, and a particle pair other than the nearest particle pair may be specified. However, when the nearest particles are used, computation is simplified, thereby reducing the amount of computation.

In the embodiment, although a case where a candidate pair is selected from the defined system P has been described, the invention is not limited thereto, and a candidate pair may be selected from the updated system Q.

In the embodiment, although a case where the system position adjustment unit 134 moves the defined system P has been described, the invention is not limited thereto, and the system position adjustment unit may move the updated system Q, instead of the defined system P, or may move both systems.

In the embodiment, although a case where the position relationship change unit 140 changes the position of each particle of the updated system Q has been described, the invention is not limited thereto, and for example, the position of each particle of the defined system P may be changed, or the position of each particle of both the defined system P and the updated system Q may be changed.

In the embodiment, although a case where the position relationship change unit 140 increases the difference vector has been described, the invention is not limited thereto, and for example, the position relationship change unit may decrease the difference vector. In this case, for example, when change in the updated system is too large and difficult to understand, the change decreases, whereby it is possible to easily understand the change.

In the embodiment, although a case where the maintenance determination unit 132 compares the computed inter-particle distance with the inter-particle distance of the candidate pair computed by the defined system particle group selection unit 130, and determines that candidate pair is a pair in which the inter-particle distance is substantially maintained before and after the update of the position when both coincide with each other within a predetermined error range has been described, the invention is not limited thereto. For example, the maintenance determination unit may compare in the inter-particle distance all the nearest particle pairs of the defined system P with the corresponding particle pair of the updated system Q and may determine a candidate pair, in which the difference is smallest, that is, the inter-particle distance is least changed, as an inter-particle distance maintaining pair.

### Reference Signs List

100: analysis device, 110: defined system acquisition unit, 114: numerical computation unit, 128: particle group specification unit, 134: system position adjustment unit, 140: position relationship change unit.

### Industrial Applicability

According to the invention, it is possible to readily capture a phenomenon generated in a system in a simulation using a particle method.

## Claims

1. An analysis device comprising:
a defined system acquisition unit configured to acquire a system including a plurality of particles defined in a virtual space as a defined system;
a numerical computation unit configured to numerically compute a governing equation, which governs the motion of each particle of the defined system, and to update the position of each particle of the defined system based on the computation result;
a particle group specification unit configured to specify a particle group, the relative position relationship of which is substantially maintained before and after the update by the numerical computation unit, from a plurality of particles in the defined system;
a system position adjustment unit configured to, when a system obtained as a result of the update by the numerical computation unit is referred to as an updated system, adjust the relative positions of the defined system and the updated system such that the particle group specified by the particle group specification unit and a particle group in the updated system corresponding to the specified particle group overlap each other; and
a position relationship change unit configured to change the relative position relationship between a particle in the defined system and a particle in the updated system corresponding to the particle in the defined system for the defined system and the updated system having the relative positions adjusted by the system position adjustment unit.

2. The analysis device according to claim 1,
wherein the system position adjustment unit performs congruence transformation for at least one of the defined system and the updated system to adjust the relative positions of the defined system and the updated system.

3. The analysis device according to claim 1 or 2,
wherein the particle group specification unit specifies a particle group, the relative distance of which is substantially maintained before and after the update by the numerical computation unit, from a plurality of particles in the defined system.

4. The analysis device according to any one of claims 1 to 3,
wherein the position relationship change unit changes the relative distance between the particle in the defined system and the particle in the updated system corresponding to the particle in the defined system in a predetermined ratio.

5. A simulation method comprising:
a step of acquiring a system including a plurality of particles defined in a virtual space as a defined system;
a step of numerically computing a governing equation, which governs the motion of each particle of the defined system;
a step of updating the position of each particle of the defined system based on the computation result;
a step of, when a system obtained as a result of the update is referred to as an updated system, adjusting the relative positions of the defined system and the updated system; and
a step of increasing the difference between the defined system and the updated system after the relative positions are adjusted.

6. A computer program which causes a computer to realize:
a function of acquiring a system including a plurality of particles defined in a virtual space as a defined system;
a function of numerically computing a governing equation, which governs the motion of each particle of the defined system, and updating the position of each particle of the defined system based on the computation result;
a function of specifying a particle group, the relative position relationship of which is substantially maintained before and after the update, from a plurality of particles in the defined system;
a function of, when a system obtained as a result of the update is referred to as an updated system, adjusting the relative positions of the defined system and the updated system such that the specified particle group and a particle group in the updated system corresponding to the specified particle group overlap each other; and
a function of changing the relative position relationship between a particle in the defined system and a particle in the updated system corresponding to the particle in the defined system for the defined system and the updated system having the adjusted relative positions.
